# EUROPEAN PATENT APPLICATION

(11) **EP 2 179 766 A1**
(43) Date of publication of application: **28.04.2010**
(21) Application number: 09173839.3
(22) Date of filing: 22.10.2009
(51) Int. Cl.: A61N 5/06

(54) **Method of treatment for dermatologic disorders**

(30) Priority: 22.10.2008 US 555994
(71) Applicant: Dusa Pharmaceuticals, Inc., Wilmington, MA 01887 (US)
(72) Inventor: Lundahl, Scott, Wilmington, MA 01887 (US); Marcus, Stuart L., Wilmington, MA 01887 (US)
(74) Representative: Hatzmann, Martin

(57) **Abstract**

A method of treating acne using lower light intensity, fewer blue light treatments, and no photodynamic or photosensitizing agents is provided.

## Description

### Field of the Invention

This invention relates to the treatment of dermatologic disorders. In particular, it relates to methods for the treatment of acne vulgaris.

### Background of the Invention

Acne vulgaris (often simply known as "acne") is one of the most common dermatologic disorders. The majority of the population suffers from acne for a portion of their lives, most often during adolescence and young adulthood. Acne is a disorder of the pilosebacious unit. While its etiology is thought to be multifactorial, it is generally believed that the microorganism *P. acnes* has a role in the cause and/or aggravation of acne. Acne is characterized by an inflammation of the pilosebacious unit and surrounding tissue. The lumen associated with the follicle can become filled with cellular byproducts of this inflammation and of the growth and metabolism of *P. acnes.* This can result in further inflammation and in the clogging of the opening of the pilosebacious unit which aggravates the condition and results in a "closed comedone." The number of acne lesions and severity of acne inflammations can vary widely between individuals. Most acne lesions can be at least temporarily sensitive or present an undesirable appearance. Some acne lesions can become so severe that they can result in disfigurement and scarring.

Acne lesions can occur almost anywhere on the body, but are most commonly found on the face and back.

There are many known treatments for acne. Topical and systemic antibiotics such as minocycline, clindamycin, and doxicycline are used to reduce the population of *P. acnes.* Some systemic antibiotics are also believed to exert an anti-inflammatory effect on acne lesions. Compounds such as benzoyl peroxide are used for their keratolytic effect on acne lesions, and may also reduce the population of *P. acnes.* Various topically and systemically administered retinoid compounds are also known to be useful in treating acne through their effects on epidermal differentiation. All of these treatments are known to have drawbacks. Antibiotics can adversely affect the beneficial microflora of the body, and their use can give rise to antibiotic resistance. Keratolytic compounds such as benzoyl peroxide are often irritating to the skin. Retinoids can be irritating to the skin when applied topically, and systemic retinoids can be teratogenic.

Exposure to light, especially at "blue" wavelengths, is also known to be useful in the treatment of acne. (Taub 2007) While the mechanism of action of light on acne lesions is not fully known or understood, it is thought that light can act on *P. acnes,* either directly or by way of action on porphyrins generated by the *P. acnes* organisms. The use of photodynamic agents such as aminolevulinic acid in conjunction with light ( as described, for example, in U.S. Pat. No. 5,955,490, issued to Kennedy et al.) is a known therapy for acne. The use of photodynamic agents has been believed to result in a more efficacious treatment of acne than light alone.

Blue light as a treatment for acne is not without drawbacks. Treatments typically use a cumulative light intensity of at least 80 J/cm², and as much as 360 J/cm². Acute photodamage can result from this. Also, it has been understood that light treatments must be repeated frequently over several weeks or even months. For example, Gold and his co-workers treated patients with 10 J/cm² twice a week for four weeks and reported that this resulted in a 36% reduction in inflammatory acne lesions. (Gold, et al. 2005) Some physicians treat acne by administering light treatments daily for 3 months. Papageorgiou followed this regimen of 90 treatments, using blue light to expose skin to cumulative light energy of 320 J/cm², and obtained a 63% reduction in inflammatory lesions. (Papageorgiou et al. 2000). Akaraphanth treated skin exhibiting acne by administering a dose of 48 J/cm² weekly for four weeks and obtained a 21% reduction in inflammatory lesions after the fourth week. (Akaraphanth 2007) Shalita and his co-workers treated patients with a 54 J/cm² dose of blue light twice a week for four weeks, and reported a 60% reduction of facial inflammatory acne lesions. (Shalita 2001) These are time-consuming treatment regimens that patients find very burdensome. Also, because these treatments are administered under the supervision of a physician in the physician's office, it is very difficult and expensive for patients to use these treatment protocols and many health insurers reject them in favor of less expensive, drug-based treatments. Many patients refuse to embark upon blue light treatment regimens for their acne because of the cost and inconvenience. The topical use photodynamic agents is an additional expense, and can result in discomfort for the patent, at times even pain.

New acne treatments that are effective, less costly, less time consuming, and subject to fewer or less serious side effects are needed.

### Summary of the Invention

A method of treating acne using lower light intensity, fewer blue light treatments, and no photodynamic or photosensitizing agents is provided.

### Detailed Description of the Invention

It has been found that, contrary to what would be expected from current knowledge, blue light treatments for acne according to the current invention are as effective as conventional blue light acne treatment regimens that use exposures to more intense light, more episodes of such exposures, and at more frequent intervals, often with photosensitizing or photodynamic agents. Consequently, the current invention makes it possible to use blue light as an effective treatment for acne in a regimen that is less expensive, easier for patients to comply with, and is subject to fewer or less severe side effects.

Acne treatments according to the present invention depart from the accepted wisdom in this field in several ways. Blue light intensities of 3 to 12 J/cm² are used no more frequently than once every 14 - 28 days for 56 - 112 days. In embodiments of this invention, patients are exposed to light at a frequency of once every 18 - 24 days for 80 - 90 days. In preferred embodiments, light intensities of 5 to 10 J/cm² are used. Preferred frequencies of treatment include every 21 days for 84 days. A treatment regimen of twice a week for four weeks is particularly useful. Acne treatments according to the present invention are made using light, without additional photosensitizers or photodynamic agents.

Blue light sources that may be used in this invention are any that are capable of generating light in the range of useful wavelengths in the range of useful intensities. The blue light used in treatments according to the present invention is of a wavelength from 350 to 450 nm, and preferably in the range of 400 to 430. The range of light intensities useful in this invention are from 3 to 12 J/cm², and in preferred embodiments, from 5 to 10 J/cm². The BLU-U® Illuminator, sold by DUSA Pharmaceuticals is a light source suitable for use in this invention.

In a first embodiment the invention thus provides a source of blue light, preferably a medical device, for use in the treatment of a human or animal body, wherein said treatment comprises repeatedly exposing skin to said light, wherein at each exposure the skin is exposed to light energy in the amount of 3 to 12J/cm², and wherein the skin is cumulatively exposed to light energy in the amount of 12 to 72J/cm², and wherein the skin is not exposed to or treated with a photosensitizer or photodynamic agent in connection with the light exposure.

In another embodiment the invention provides a source of blue light, preferably a medical device, for use in the treatment of acne vulgaris, wherein said treatment comprises repeatedly exposing skin of a patient exhibiting acne vulgaris to said light, wherein at each exposure the skin is exposed to light energy in the amount of 3 to 12 J/cm², and
wherein the skin in cumulatively exposed to light energy in the amount of 12 to 72 J/cm², and wherein the skin is not exposed to or treated with a photosensitizer or photodynamic agent in connection with the light exposure.

Preferably, a source of blue light for use according to the invention is provided wherein the light is of a wavelength of between 350 to 450 nm, more preferably of between 400 to 430 nm.

In another preferred embodiment, a source of blue light for use according to the invention is provided, wherein at each exposure the skin is exposed to light energy in the amount of 3 to 12 J/cm², more preferably in the amount of 5 to 10 J/cm².

In yet another preferred embodiment, a source of blue light for use according to the invention is provided, wherein the exposure to light is repeated at intervals of from 14 to 28 days, more preferably at intervals of from 18 to 24 days.

Alternatively, or additionally, a source of blue light for use according to the invention is provided wherein the exposure to light is repeated at intervals of from 56 to 112 days, more preferably at intervals of from 80 to 90 days.

In yet another preferred embodiment, a source of blue light for use according to the invention is provided, wherein the source of blue light is accompanied by an instruction leaflet for such use.

The present invention further provides an anti-acne agent for use in the treatment of acne vulgaris, wherein said treatment further comprises repeatedly exposing skin of a patient exhibiting acne vulgaris to blue light, wherein at each exposure the skin is exposed to light energy in the amount of 3 to 12 J/cm², and
wherein the skin is cumulatively exposed to light energy in the amount of 12 to 72 J/cm², and wherein the skin is not exposed to or treated with a photosensitizer or photodynamic agent in connection with the light exposure. A use of an anti-acne agent for the manufacture of a medicament for treating acne vulgaris, wherein the treatment further comprises repeatedly exposing skin of a patient exhibiting acne vulgaris to blue light, wherein at each exposure the skin is exposed to light energy in the amount of 3 to 12 J/cm², and
wherein the skin is cumulatively exposed to light energy in the amount of 12 to 72 J/cm², and wherein the skin is not exposed to or treated with a photosensitizer or photodynamic agent in connection with the light exposure, is also provided herewith.As said before, the light is preferably of a wavelength of between 350 to 450 nm, more preferably of between 400 to 430 nm.

At each exposure the skin is preferably exposed to light energy in the amount of 3 to 12 J/cm², more preferably in the amount of 5 to 10 J/cm².

The exposure to light is preferably repeated at intervals of from 14 to 28 days, more preferably at intervals of from 18 to 24 days.

Alternatively, the exposure to light is repeated at intervals of from 56 to 112 days, more preferably at intervals of from 80 to 90 days. In a further preferred embodiment, an anti-acne agent for use according to the invention is provided, accompanied by an instruction leaflet for such use.

In yet another preferred embodiment, a use according to the invention is provided, wherein said medicament is accompanied by an instruction leaflet for such use.

The invention further provides a method of treating acne vulgaris in a patient in need of such treatment, comprising the step of repeatedly exposing the patient's skin exhibiting acne vulgaris to light, wherein the light is blue light, and wherein at each exposure the skin is exposed to light energy in the amount of 3 to 12 J/cm², and wherein the skin is cumulatively exposed to light energy in the amount of 12 to 72 J/cm², wherein the skin exhibiting acne vulgaris is not exposed to or treated with a photosensitizer or photodynamic agent in connection with the light exposure.

In a preferred embodiment, a method according to the invention is provided wherein the light is of a wavelength of between 350 to 450 nm, more preferably between 400 to 430 nm.

In another preferred embodiment, a method according to the invention is provided, wherein at each exposure the skin is exposed to light energy in the amount of 3 to 12 J/cm², more preferably in the amount of 5 to 10 J/cm².

In another preferred embodiment, a method according to the invention is provided, wherein the exposure to light is repeated at intervals of from 14 to 28 days, more preferably at intervals of from 18 to 24 days.

In yet another preferred embodiment, a method according to the invention is provided, wherein the exposure to light is repeated over a period of from 56 to 112 days, more preferably a period of from 80 to 90 days.

### Examples

### Example One

A group of 66 patients with moderate to severe acne having at least 20 inflammatory acne lesions were enrolled in a multi-center study of the effectiveness of this invention. None had recently used topical or systemic acne treatments, or were to use artificial tanning light devices during the study. A baseline dermatologic evaluation of each patient was made, including a baseline measurement of the number of inflammatory acne lesions. Each patient was given a blue light treatment of 5 J/cm² using a BLU-U® Illuminator on days 1, 21, 42, and 63. Each treatment was accompanied by the topical application of a placebo solution. The number of inflammatory lesions in each patient was measured two days after each light treatment, and at follow-up evaluations three weeks and six weeks after the last light treatment. The resulting data is presented in Table 1. It can be seen that this treatment regimen resulted in a 31% reduction in inflammatory lesions immediately after the last treatment, and the patient's acne continued to resolve itself, leading to a 36% reduction at 3 weeks post treatment, and 39% at 6 week post treatment.

**Table 1**

| Evaluation Time | Mean Change in Number of Inflammatory Lesions | Mean Percent Change from Baseline |
|---|---|---|
| Baseline | 0 | 0 |
| Day 23 | -9 | -19 |
| Day 44 | -17 | -33 |
| Day 67 | -17 | -31 |
| 3 Weeks After Final Treatment | -20 | -36 |
| 6 Weeks After Final Treatment | -22 | -39 |

### Example Two

A group of 67 patients with moderate to severe acne having at least 20 inflammatory acne lesions were enrolled in a multi-center study of the effectiveness of this invention. None had recently used topical or systemic acne treatments, or were to use artificial tanning light devices during the study. A baseline dermatologic evaluation of each patient was made, including a baseline measurement of the number of inflammatory acne lesions. Each patient was given a blue light treatment of 10 J/cm² using a BLU-U® Illuminator on days 1, 21, 42, and 63. Each treatment was accompanied by the topical application of a placebo solution. The number of inflammatory lesions in each patient was measured two days after each light treatment, and at follow-up evaluations three weeks and six weeks after the last light treatment. The resulting data is presented in Table 2. It can be seen that this treatment regimen resulted in a 38% reduction in inflammatory lesions immediately after the last treatment, and the patient's acne continued to resolve itself, leading to a 38% reduction at 3 weeks post treatment, and 43% at 6 week post treatment.

**Table 2**

| Evaluation Time | Mean Change in Number of Inflammatory Lesions | Mean Percent Change from Baseline |
|---|---|---|
| Baseline | 0 | 0 |
| Day 23 | -9 | -18 |
| Day 44 | -15 | -28 |
| Day 67 | -20 | -38 |
| 3 Weeks After Final Treatment | -21 | -38 |
| 6 Weeks After Final Treatment | -23 | -43 |

The results of these two examples show that the invention is a very effective treatment of acne, and that the results of this treatment persist well after treatment is concluded. These results are comparable to the results obtained from conventional blue light treatments for acne that use more frequent light treatments, more intense light treatments, light treatment in conjunction with photodynamic or photosensitizing agents, or combinations of them.

However, treatment according to the present invention is clearly superior to the prior art methods in that it is cheaper to administer, more easily complied with and completed, and less susceptible to undesirable side effects.

## Claims

1. A source of blue light for use in the treatment of a human or animal body, wherein said treatment comprises repeatedly exposing skin to said light, wherein at each exposure the skin is exposed to light energy in the amount of 3 to 12 J/cm², and
wherein the skin is cumulatively exposed to light energy in the amount of 12 to 72 J/cm², and wherein the skin is not exposed to or treated with a photosensitizer or photodynamic agent in connection with the light exposure.

2. A source of blue light for use in the treatment of acne vulgaris, wherein said treatment comprises repeatedly exposing skin of a patient exhibiting acne vulgaris to said light, wherein at each exposure the skin is exposed to light energy in the amount of 3 to 12 J/cm², and
wherein the skin is cumulatively exposed to light energy in the amount of 12 to 72 J/cm², and wherein the skin is not exposed to or treated with a photosensitizer or photodynamic agent in connection with the light exposure.

3. A source of blue light according to claim 1 or 2, wherein the source of blue light is a medical device.

4. An anti-acne agent for use in the treatment of acne vulgaris, wherein said treatment further comprises repeatedly exposing skin of a patient exhibiting acne vulgaris to blue light, wherein at each exposure the skin is exposed to light energy in the amount of 3 to 12 J/cm², and
wherein the skin is cumulatively exposed to light energy in the amount of 12 to 72 J/cm², and wherein the skin is not exposed to or treated with a photosensitizer or photodynamic agent in connection with the light exposure.

5. A source of blue light according to any one of claims 1 - 3, or an agent according to claim 4, wherein the light is of a wavelength of between 350 to 450 nm.

6. A source of blue light, or an agent according to claim 5, wherein the light is of a wavelength of between 400 to 430 nm.

7. A source of blue light, or an agent according to any one of claims 1 - 6, wherein at each exposure the skin is exposed to light energy in the amount of 3 to 12 J/cm².

8. A source of blue light, or an agent according to claim 7, wherein at each exposure the skin is exposed to light energy in the amount of 5 to 10 J/cm².

9. A source of blue light, or an agent according to any one of claims 1 - 8, wherein the exposure to light is repeated at intervals of from 14 to 28 days.

10. A source of blue light, or an agent according to claim 9 wherein the exposure to light is repeated at intervals of from 18 to 24 days.

11. A source of blue light, or an agent according to any one of claims 1 - 8, wherein the exposure to light is repeated at intervals of from 56 to 112 days.

12. A source of blue light, or an agent according to claim 11, wherein the exposure to light is repeated at intervals of from 80 to 90 days.

13. A source of blue light, or an agent according to any one of claims 1 - 12, accompanied by an instruction leaflet for such use.
